# EUROPÄISCHE PATENTSCHRIFT

(11) **EP 1 467 682 B1**
(45) Veröffentlichungstag und Bekanntmachung des Hinweises auf die Patenterteilung: **25.05.2005**
(21) Anmeldenummer: 02799068.8
(22) Anmeldetag: 20.12.2002
(51) Int. Cl.: A61F 2/40

(54) **SCHULTERPROTHESE**
SHOULDER PROSTHESIS
PROTHESE D'EPAULE

(30) Priorität: 21.12.2001 DE 20120703 U
(43) Veröffentlichungstag der Anmeldung: 20.10.2004
(73) Patentinhaber: Mathys AG Bettlach, 2544 Bettlach (CH)
(72) Erfinder: BLATTER, Georges, CH-9000 St. Gallen (CH); SCHWÄGERL, Wolfgang, A-1010 Wien (AT); ZENZ, Peter, A-3003 Gablitz (AT); DALLMANN, Frank, 04626 Schmölln (DE); GLIEN, Wilfried, 07639 Bad Klosterlausnitz (DE); SALOMON, Dirk, 04626 Jonaswalde (DE); IRLENBUSCH, Ulrich, 99310 Arnstadt (DE)
(74) Vertreter: Körfer, Thomas
(86) Internationale Anmeldenummer: PCT/EP2002/014634
(87) Internationale Veröffentlichungsnummer: WO 2003/053280

(56) Entgegenhaltungen:
- WO-A-01/82843
- US-A- 5 336 268
- US-A- 5 358 526
- US-A- 5 800 560

## Beschreibung

Die Erfindung betrifft eine Schulterprothese mit einem Schaft, mit einer Kopfkalotte und mit einem Mittelteil, das am proximalen Ende des Schaftes an verschiedenen Positionen entlang einer ersten linearen Richtung befestigbar ist und an dem die Kopfkalotte befestigbar ist.

Eine solche Schulterprothese ist aus EP-A 0 953 321 bekannt. Das Mittelteil hat dabei nach außen abstehende Rippen, mittels denen es in einer Ausnehmung am proximalen Ende des Schaftes in verschiedenen lateral-medial gestaffelten Positionen einsetzbar ist. Die Verbindung des Mittelteils mit dem Schaft erfolgt durch eine Presspassung und die Kopfkalotte hat eine Ausnehmung von gleichbleibendem Querschnitt, mittels der sie auf das Mittelteil aufgesetzt wird. Das Mittelteil ist abgewinkelt, wodurch verschiedene Inklinationen und Retroversionswinkel einstellbar sind.

Aus US-A 5 961 555 ist eine Schulterprothese bekannt, bei der das Mittelteil auf einen Konus am proximalen Ende des Schaftes aufsetzbar ist und die Kopfkalotte mittels einer konischen Schwalbenschwanzführung festgespannt wird. Abgesehen davon, dass die Kopfkalotte beim Festspannen am Mittelteil in anterior-posteriorer Richtung verschoben wird, ist die Kopfkalotte nicht gegenüber dem Schaft einstellbar.

Aus EP-A 0 549 480, EP-A 0 599 429, FR-A 2 721 200 und US-A 6 197 062 sind Schulterprothesen bekannt, bei denen das Mittelteil an beiden Enden konisch ausgebildet ist und dadurch mit dem Schaft und der Kopfkalotte verbindbar ist. Die Achsen der beiden konischen Enden sind zueinander abgewinkelt und/oder gegeneinander versetzt, wodurch eine lateral-mediale und posterior-anteriore Einstellung der Kopfkalotte und ggf. des Retroversionswinkels gegenüber dem Schaft möglich ist. Eine ähnliche Schulterprothese ist auch aus DE-A 198 41 612 bekannt, wobei das Mittelteil scheibenförmig ist und auf beiden Seiten axiale, gegeneinander versetzte Zapfen aufweist.

Aus EP-A 0 679 375 ist eine Schulterprothese bekannt, bei der das Mittelteil einen zylindrischen Teil aufweist, dessen Achse parallel zur Schaftachse verläuft und der am proximalen Ende des Schaftes mit einstellbarer Drehlage festspannbar ist. Von diesem zylindrischen Teil steht ein konischer Teil ab, auf den die Kopfkalotte aufsetzbar ist.

Aus EP-A 0 712 617, US-A 6 228 120 und US-A 6 206 925 sind Schulterprothesen bekannt, bei denen das Mittelteil mittels eines Kugelgelenks gegenüber dem Schaft einstellbar ist, wobei die konkave Fläche des Kugelgelenks im Schaft ausgebildet ist. Bei der aus EP-A 0 715 836 bekannten Schulterprothese ist die konkave Fläche des Kugelgelenks in der Kopfkalotte ausgebildet und die Gelenkkugel am proximalen Schaftende angesetzt.

Aus DE-A-195 48 154 und DE-U-299 18 669 sind Schulterprothesen bekannt, bei denen das Mittelteil am proximalen Ende des Schaftes in Richtung der Schaftachse einstellbar sind, wodurch der Prothesenschaft in seiner effektiven Länge veränderbar ist.

Aus DE-U-93 12 218 ist eine Schulterprothese bekannt, bei der der Abstand zwischen Kopfkalotte und Schaft dadurch veränderbar ist, dass die Kopfkalotte mit einem axialen oder achsparallelen Gewindeabschnitt versehen ist, der am proximalen Ende des Schaftes in eine Gewindebohrung einschraubbar ist. Mittels einer quer dazu einschraubbaren Sicherungschraube ist die Kopfkalotte fixierbar.

Prothesengemäß dem Oberbegriff von Anspruch 1 sind aus den Dokumenten US-A-5 336 268 und US-A-5 800 560 bekannt.

Der Erfindung liegt die Aufgabe zugrunde, eine Schulterprothese zu schaffen, die besonders einfach aufgebaut ist und bei der die Kopfkalotte praktisch stufenlos gegenüber dem Schaft verstellbar ist.

Erfindungsgemäß wird diese Aufgabe dadurch gelöst, dass das Befestigungselement in einer Führungsbohrung in dem schaft verschiebbar geführt ist und dass das Mittelteil durch ein Fenster in der wand der Führungsbohrung gegen das Befestigungselement spannbar ist, wodurch das Befestigungselement und das Mittelteil fixert werden.

Vorzugsweise ist das Befestigungselement am proximalen Ende des Schaftes in einer Führung linear einstellbar, vorzugsweise in medial-lateraler Richtung. Im Allgemeinen liegt die Richtung der Einstellbarkeit in der Resektionsebene oder in einer dazu geringfügig parallel verstetzten Ebene.

Die Resektionsebene liegt üblicherweise in einem Winkel von 40° bis 50° zur Schaftachse, d.h. die Normale der Resektionsebene liegt unter einem Winkel von 130° bis 140° zur Schaftachse. Gelenkimplantationen werden in aller Regel so durchgeführt, dass die Verbindungsfläche des Implantats möglichst genau mit der Resektionsfläche übereinstimmt. Die Verbindungsfläche ist im vorliegenden Fall die Fläche am proximalen Ende des Schaftes, an der die Kopfkalotte im Idealfall anliegt.

Vorzugsweise wird das Mittelteil mittels eines Gewindebolzens festgespannt, der durch eine Schulterbohrung des Mittelteils geht und in das Befestigungselement eingeschraubt wird, wodurch zugleich das Befestigungselement in seiner Führung fixiert wird.

Durch diese Maßnahmen ist die Kopfkalotte stufenlos in der ersten Richtung gegenüber dem Schaft einstellbar. Um die Kopfkalotte zweidimensional gegenüber dem Schaft verstellbar zu machen, ist vorzugsweise das Mittelteil gegenüber dem Befestigungselement in einer zweiten Richtung stufenlos einstellbar. Die zweite Richtung verläuft unter einem Winkel zu der ersten Richtung und ist vorzugsweise etwa rechtwinklig zur ersten Richtung.

Die Verstellbarkeit des Mittelteils gegenüber dem Befestigungselement kann dadurch erreicht werden, dass die Schulterbohrung im Mittelteil eine in anterior-posteriore Richtung zeigende Langloch-Schulterbohrung ist.

Die Kopfkalotte ist dadurch stufenlos in zwei Richtungen gegenüber dem Schaft einstellbar, wobei die Einstellung intraoperativ erfolgt und korrigierbar ist. Die Reproduktion von Inklination und Retrotorsion erfolgt hierbei nicht über eine Ausrichtung der Kopfkalotte gegenüber dem Schaft, sondern wird über Resektionsinstrumente erreicht und ist daher bei der erfindungesgemäßen Schulterprothese vorgegeben.

In einer besonders bevorzugten Ausgestaltung der Erfindung weist der Schaft am proximalen Ende eine Verbindungsfläche auf, die unter einem Winkel von etwa 40° zur Schaftachse liegt und die im Allgemeinen mit der Resektionsebene zusammenfällt oder in geringem Abstand parallel zu ihr liegt. Das Befestigungselement ist ein bolzenförmiger Gleitstein, der in einer Führungsbohrung verschiebbar ist, die in geringem Abstand unterhalb der Verbindungsfläche vorgesehen ist und ein proximales Fenster in der Verbindungsfläche hat. Der Gleitstein weist eine querverlaufende Gewindebohrung auf. Das Mittelteil ist mittels eines Gewindebolzens, der in die Querbohrung des Gleitsteins eingeschraubt wird, gegen die Verbindungsfläche spannbar. Die Verbindungsfläche und die an ihr anliegende Unterseite des Mittelteils können geriffelt sein, um einen formschlüssigen Eingriff zu erzielen. Statt der Riffelung kann auch eine Aufrauhung vorgesehen sein. Grundsätzlich ist jede form- und/oder kraftschlüssige Verbindung möglich.

Das Mittelteil hat eine leicht konische Außenseite und die Kopfkalotte hat eine entsprechend konische Ausnehmung, wodurch die Kopfkalotte durch einfaches Aufstecken auf das Mittelteil mit diesem verbindbar ist. Die Verbindung kann auch durch Formschluss, z.B. Schnappelemente, oder durch Formkraftschluss, z.B. eine Gewindeverbindung, erfolgen.

Diese Verbindungsmittel können zentriert, einfach-exzentrisch oder doppel-exzentrisch sein. Bei einer zentrierten Ausbildung ist die Schulterbohrung des Mittelteils zentriert zur Außenseite des Mittelteils und ist auch die Ausnehmung oder das sonstige Verbindungsmittel zentriert in der Kopfkalotte vorgesehen.

Bei einer einfach-exzentrischen Ausbildung ist die Schulterbohrung des Mittelteils zentriert und ist die konische Ausnehmung oder das sonstige Verbindungsmittel exzentrisch in der Kopfkalotte ausgebildet. Durch Ändern der Drehlage der Kopfkalotte auf dem Mittelteil kann der Mittelpunkt der Kopfkalotte gegenüber dem Schaftende auf einer Kreisbahn eingestellt werden, deren Radius gleich der Exzentrizität ist. In Verbindung mit der Einstellung des Mittelstücks entlang der ersten Richtung ergibt sich dadurch eine stufenlose Einstellbarkeit der Kopfkalotte gegenüber dem Schaftende in zwei Dimensionen.

Es besteht auch die Möglichkeit, die Kopfkalotte zentriert auszubilden und die Schulterbohrung exzentrisch in dem Mittelteil vorzusehen. Allgemein wird bei einfach-exzentrischen Ausbildung jedoch die vorausgehend erwähnte Möglichkeit gewählt, bei der nur die Kopfkalotte exzentrisch ausgebildet ist.

Bei einer doppel-exzentrischen Ausbildung ist sowohl die Schulterbohrung des Mittelteils exzentrisch zur Außenseite des Mittelteils angeordnet als auch die Ausnehmung oder das sonstige Verbindungsmittel exzentrisch in der Kopfkalotte vorgesehen. Die doppelte Exzentrizität ermöglicht bereits für sich eine stufenlose Verstellung der Kopfkalotte gegenüber dem Schaftende innerhalb einer Kreisfläche, deren Radius der Summe der beiden Exzentrizitäten entspricht. Zusätzlich kann die Kopfkalotte dabei noch in der ersten Richtung stufenlos eingestellt werden.

Vorzugsweise verfügt der Schaft am proximalen Ende über eine oder mehrere Finnen mit Bohrungen zur Befestigung von Sehnen und Knochenbruchstücken.

In Abhängigkeit von den anatomischen Voraussetzungen kann die Kopfkalotte eine Form zwischen einer Halbkugel und einem sehr flachen Kugelabschnitt mit einem Mittelpunktswinkel von bspw. 90° haben.

Die einzelnen Teile der Schaftprothese können aus den für Endoprothesen üblichen Materialien hergestellt werden, insbesondere aus Keramik, Titanlegierungen oder Kobalt-Chrom-Legierungen oder aus unverstärktem oder faserverstärktem Kunststoff.

Ausführungsbeispiele der Erfindung werden nachfolgend anhand der Zeichnung erläutert. Es zeigen:
- Fig. 1: ein Beispiel einer Schulterprothese in einer räumlichen Explosionsdarstellung;
- Fig. 2: die Schulterprothese von Fig. 1 von anterior bzw. posterior in einer auseinandergezogenen Darstellung;
- Fig. 3: einen Schnitt nach A-B von Fig. 2,
- Fig. 4: die Schulterprothese von Fig. 1 im zusammengebauten Zustand im Längsschnitt,
- Fig. 5: eine räumliche Darstellung eines zentrischen Mittelteils und einer Kopfkalotte mit exzentrisch angeordneter Ausnehmung,
- Fig. 6: ein Ausführungsbeispiel der erfindungsgemäßen Schulterprothese in einer räumlichen Explosionsdarstellung,
- Fig. 7: die Schulterprothese von Fig. 1 von anterior bzw. posterior in einer auseinandergezogenen Darstellung,
- Fig. 8: einen Schnitt nach A-A von Fig. 7,
- Fig. 9: die Schulterprothese von Fig. 6 im zusammengebauten Zustand im Längsschnitt und
- Fig. 10: in einer räumlichen Darstellung ein zentrisches Mittelteil und eine Kopfkalotte mit exzentrisch angeordneter Ausnehmung des Ausführungsbeispiels von Fig. 6.

Die Schulterprothese aller Ausführungsbeispiele besteht aus einem Schaft 10, einem Gleitstein 20 als Befestigungselement, einem Mittelteil 30, einem Gewindebolzen 40 und einer Kopfkalotte 50. Der Schaft 10 verbreitert sich zu seinem proximalen Ende 12 hin und endet dort in einer Verbindungsfläche 14, die entsprechend der Resektionsebene unter einem Winkel von etwa 40° oder 45° zur Längsachse des Schaftes 10 verläuft.

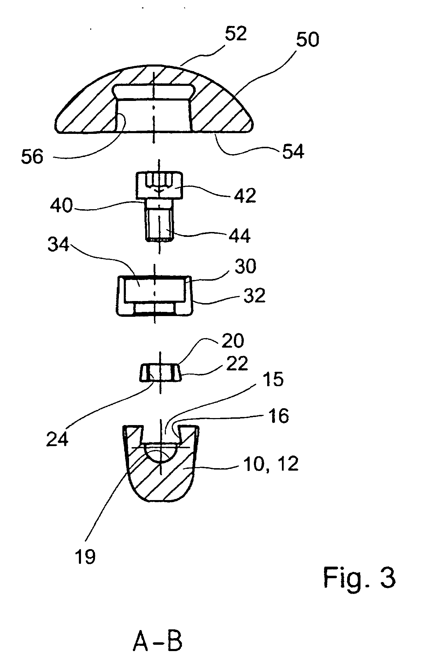

Bei einem Beispiel gemäß dem Stand der Technik wird der Gleitstein 20 in einer Schwalbenschwanzführung 15 geführt, wozu in der Verbindungsfläche 14 eine sich nach innen erweiternde Nut 16 ausgebildet ist und zwei seitliche Führungsflächen 22 des insgesamt etwa quadratischen Gleitsteins 20 entsprechend geneigt sind (Fig. 3), so dass der Gleitstein 20 in der Nut 16 in lateralmedialer Richtung verschiebbar geführt wird. In der Verbindungsfläche bleiben zu beiden Seiten der Nut 16 Ränder 18 stehen.

Das Mittelteil 30 hat eine kegelstumpfförmige Außenseite 32 und eine axiale Langloch-Schulterbohrung 34, wobei die längere Abmessung der Schulterbohrung 34 anterior-posterior gerichtet ist. In die Schulterbohrung 34 wird der Gewindebolzen 40 eingesetzt und in eine Gewindebohrung 24 des Gleitsteins 20 eingeschraubt. Der Gewindebolzen 40 hat einen Kopf 42 mit einem Innensechskant und einen Gewindeschaft 44 und der Kopf des Gewindebolzens 40 wird vollständig in der Schulterbohrung 34 aufgenommen. Durch Festziehen des Gewindebolzens 40 wird das Mittelteil 30 gegen die äußeren Ränder 18 der Schwalbenschwanzführung 15 gespannt. Das Ende des Gewindebolzens 40 reicht dabei bis in eine Aussparung 19 im Boden der Schwalbenschwanzführung 15.

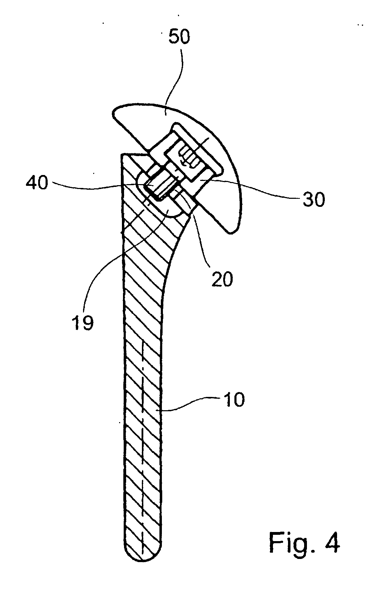

Die Kopfkalotte 15 entspricht in ihrer äußeren Form etwa einem Kugelabschnitt mit einem Mittelpunktswinkel von etwa 90°. Sie hat dementsprechend eine gewölbte Oberseite 52 und eine ebene Unterseite 54. In der Unterseite 54 ist eine sich konisch verengende Ausnehmung 56 vorgesehen, so dass die Kopfkalotte 50 mit Presspassung auf das Mittelteil 30 aufgesetzt werden kann. Die Ausnehmung 56 hat dabei eine solche Tiefe, dass sie das Mittelteil 30 vollständig aufnimmt. Wie Fig. 4 zeigt, liegt die Unterseite 54 der Kopfkalotte 50 im zusammengebauten Zustand der Schulterprothese praktisch auf der Verbindungsfläche 14 und damit auf der Resektionsebene auf.

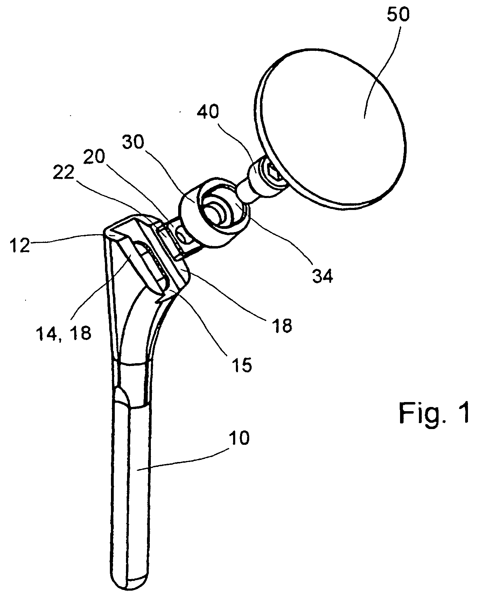

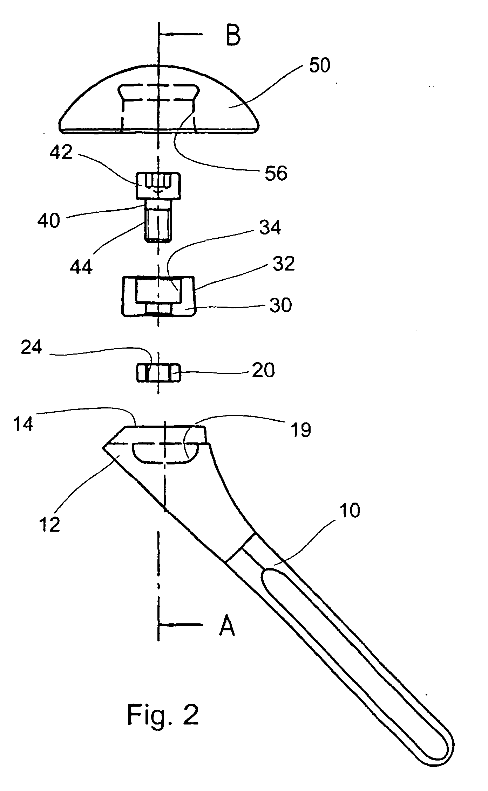

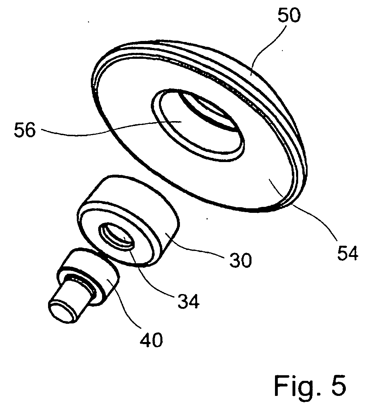

Fig. 5 zeigt ein Beispiel einer Schulterprothese, ähnlich dem der Fig. 1 bis 4, wobei jedoch die Schulterbohrung 34 kreisförmig ist. Das Mittelteil 30 kann daher nicht anterior-posterior eingestellt werden. Um dennoch eine stufenlose Verstellbarkeit in zwei Dimensionen zu erreichen, ist die Ausnehmung 56 in der Unterseite 54 der Kopfkalotte 50 exzentrisch angeordnet. Der Mittelpunkt der Kopfkalotte 50 kann daher auf einer Kreislinie um die Längsachse des Mittelteils 30 positioniert werden. In Verbindung mit der medial-lateralen Einstellbarkeit des Mittelteils 30 an dem proximalen Ende 12 des Schafts 10 erhält man dadurch ebenfalls eine stufenlose Einstellbarkeit innerhalb einer ausreichend großen Fläche, um den anatomischen Gegebenheiten des Einzelfalls entsprechen zu können.

Der Zusammenbau der Endoprothese kann vor oder nach dem Einsetzen des Schaftes 10 in den Humeruskanal erfolgen, wobei der Gewindebolzen 40 durch das Mittelteil 30 hindurch zunächst lose in den Gleitstein 20 eingeschraubt wird. Das Befestigungselement 20 kann sich dabei bereits in der Nut 16 am proximalen Ende 12 des Schaftes 10 befinden. Andererseits kann der Gleitstein 20 auch in lose mit dem Mittelteil 30 verbundenen Zustand in die Nut 16 eingesetzt werden. Der Schaft 10 wird dann positioniert und das Mittelteil 30 wird so fixiert, dass die Kopfkalotte 50 möglichst genau der anatomischen Situation entspricht.

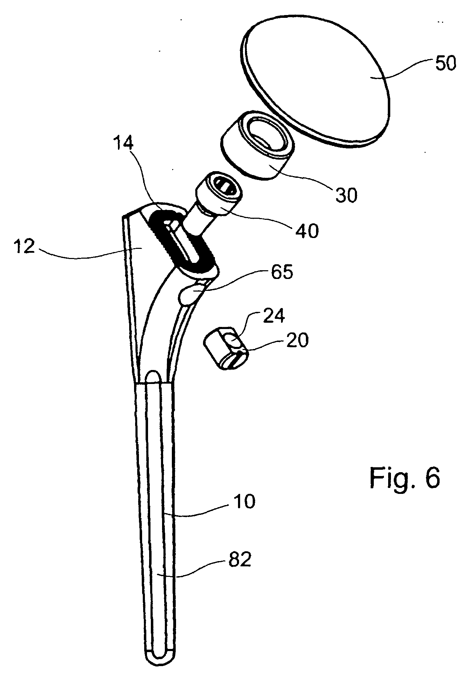

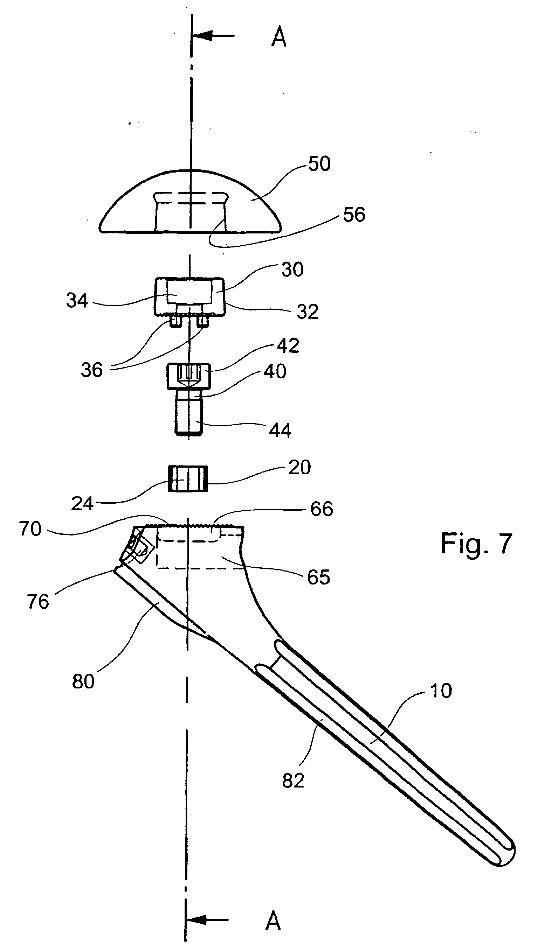

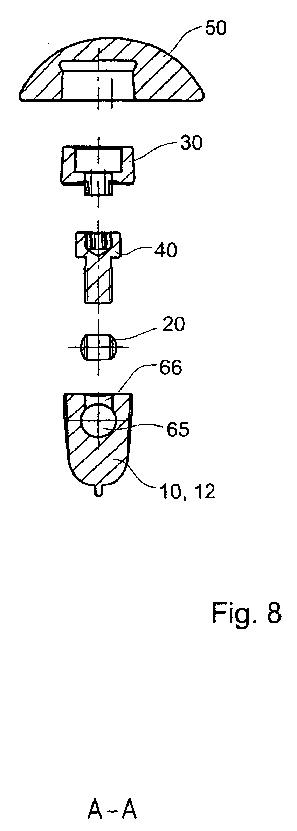

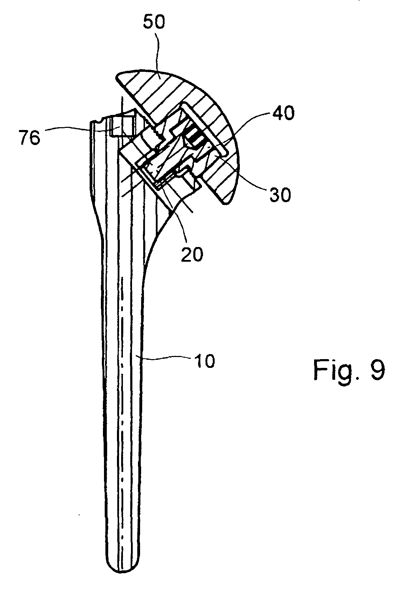

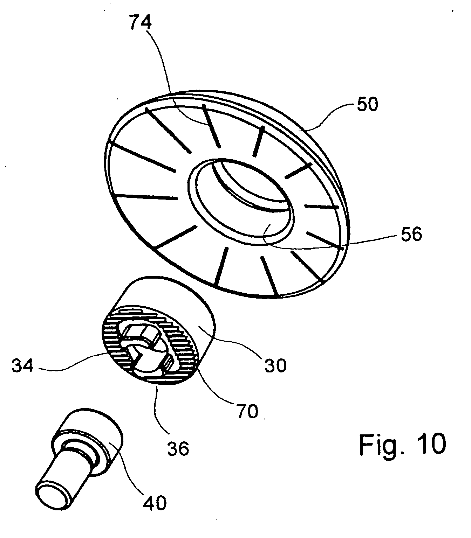

Bei einem Ausführungsbeispiel der erfindungsgemäßen Schulterprothese (Fig. 6 bis 10) ist das Befestigungselement ein bolzenförmiger Gleitstein 20, wobei die Gewindebohrung 24 quer durch den Gleitstein 20 verläuft. Der Gleitstein 20 wird in einer Führungsbohrung 65 im Schaft 10 geführt, die von medial in den Schaft 10 eingebracht ist und deren Achse parallel zur proximalen Verbindungsfläche 14 des Schaftes 10 verläuft. Der Gleitstein 20 ist in der Führungsbohrung in medial-lateraler Richtung verschiebbar.

In die proximale Verbindungsfläche 14 des Schaftes 10 ist ein Fenster 66 bis zu der Führungsbohrung 65 eingebracht. Der Gewindebolzen 40 wird so wie beim ersten Ausführungsbeispiel durch das Mittelteil 30 hindurchgesteckt. Bei dem vorliegenden dritten Ausführungsbeispiel wird er dann durch das Fenster 66 hindurchgeführt und in die Gewindebohrung 24 des Befestigungselements 20 eingedreht. Mittels des Gewindebolzens 40 wird dadurch das Mittelteil 30 gegen den Gleitstein 20 und damit gegen die Verbindungsfläche 14 des Schafts 10 gespannt. Die Länge des Fensters 66 definiert den medial-lateralen Verschiebeweg des Mittelteiles 30 gegenüber dem Schaft 10.

Das Mittelteil 30 hat eine kegelstumpfförmige Außenseite und eine konzentrische Durchgangsbohrung 34 mit einer Sacklochbohrung, die von der proximalen Oberseite oder Abschlussfläche ausgeht. In die Sacklochbohrung wird der Gewindebolzen 40, wie erwähnt, eingesetzt und in die Gewindebohrung 24 des Gleitsteins 20 eingeschraubt, der vorab von medial in die Führungsbohrung 65 eingeführt wurde. Der Gewindebolzen 40 hat einen Kopf mit einem Innensechskant und einen Gewindeschaft und der Kopf der Schraube wird vollständig in der Sacklochbohrung des Mittelteiles 30 aufgenommen.

Eine Rillenstruktur an der verbindungsfläche 14 des Schaftes 10 und an der Unterseite des Mittelteiles 30 gewährleistet einen Form-/Kraftschluss als zusätzliche Sicherung des Verbundes gegen Lösen und Mikrobewegungen. Die Rillen 70 sind im 1 mm-Raster angeordnet. Somit wird eine definierte Verstellung der Kopfkalotte 50 in medial-lateraler Richtung sichergestellt. Von der Unterseite des Mittelteiles 30 stehen Führungsnasen 36 hervor, welche in das Fenster 66 des Schaftes 10 eingreifen. Somit wird das Einrasten und die Ausrichtung des Mittelteiles 30 zum Schaft 10 entsprechend der Rillenstruktur gewährleistet.

Die Kopfkalotte 50 entspricht der des zweiten Ausführungsbeispiels. Die Achse der Konusausnehmung 56 liegt um einen Betrag von 3 mm (Exzentrizität) versetzt zur zentrischen Achse der Kopfkalotte 50. Die Kopfkalotte 50 ist damit auf einer definierten Exzenterbahn um die Achse des Mittelteiles 30 rotierbar.

An der Unterseite der Kopfkalotte sind Markierungen 74 und Beschriftungen für die Exzenterstellungen vorgesehen, vorzugsweise in der Art und Weise einer Uhr-Beschriftung in zwölf Einheiten. Die Eintrittsöffnung der Führungsbohrung 65 kann mittels eines Verschlusses verschlossen werden.

Durch die Kombination der Kopfexzentrizität mit dem Verschiebemechanismus in medial/lateraler Richtung ist somit jede beliebige Position der Kopfkalotte zum Schaft auf der Resektionsebene einstellbar (sog. mediodorsaler Offset). Die Einstellbereiche betragen jeweils ± 3 mm. Damit erhält man eine stufenlose Einstellbarkeit innerhalb einer ausreichend großen Fläche, um den anatomischen Gegebenheiten des Einzelfalls entsprechen zu können.

Die Konussteigung des Mittelteiles und Kopfes entspricht der bei Implantatkonen eingesetzten Steigung von 1 : 10.

Am proximalen Schaftende sind eine zentrale Gewinde-Sacklochbohrung 76 zur Verspannung mit dem Setzinstrument sowie zwei Sacklochbohrungen zur Rotationssicherung des Setzinstrumentes angeordnet.

Der Schaft besitzt proximal/lateral eine Finne 80 zur Rotationssicherung. Bei der zementfreien Schaftauslegung können solche Finnen auch an den anterioren/posterioren proximalen Seitenflächen vorgesehen werden. Ebenfalls zur Rotationssicherung im Zementmantel dienen Zementnuten 82 im distalen Schaftbereich. In dieser Ausführung sind sie 4 mal um die Schaftachse rotiert.

Das Modul aus Schaft 10, Mittelteil 30, Gleitstein 20 und Schraube 40 wird vormontiert und unverspannt in einer Sterilverpackung geliefert. Es ist auch möglich, bei bereits im Schaft 10 befindlichem Gleitstein 20 das Mittelteil 30 mit Schraube 40 nach Implantation des Schaftes zu montieren und im Revisionsfall das Mittelteil 30 zu wechseln. Um den Gleitstein 20 für das Eindrehen der Schraube 40 ausrichten zu können, ist in der Stirnfläche des Gleitsteins 20 eine Quernut 84 geschnitten.

Während bei dem Beispiel der Figuren 1 bis 4 der Zugang von proximal/lateral erfolgt, ist ein besonderer Vorteil des Ausführungsbeispiels der Figuren 6 bis 10, dass der Zugang von medial erfolgt. Ferner wird durch die Führungsnasen 36 in Verbindung mit der Rasterung eine besonders zuverlässige Fixierung des Mittelteils erreicht. Die Einstellung des Mittelteils ist bei dem Ausführungsbeispiel der Figuren 6 bis 10 nicht nur interoperativ am implantierten Schaft möglich, sondern auch mittels einer Test-Raspel (Raspel mit Einstellmechanismus) und externer Übertragung der Einstellparameter auf das Implantat möglich.

### Bezugszeichenliste

- 10: Schaft
- 12: proximales Ende
- 14: Verbindungsfläche
- 15: Schwalbenschwanzführung
- 16: Nut
- 18: Ränder
- 19: Aussparung
- 20: Gleitstein
- 22: Führungsflächen
- 24: Gewindebohrung
- 30: Mittelteil
- 32: Außenseite
- 34: Schulterbohrung
- 36: Führungsnasen
- 40: Gewindebolzen
- 42: Kopf
- 44: Gewindeschaft
- 50: Kopfkalotte
- 52: Oberseite
- 54: Unterseite
- 56: Ausnehmung
- 65: Führungsbohrung
- 66: Fenster
- 70: Rillen
- 74: Markierungen
- 76: Sacklochbohrung
- 80: Finne
- 82: Zementnuten

## Patentansprüche

1. Schulterprothese
- mit einem Schaft (10),
- mit einer Kopfkalotte (50) und
- mit einem Mittelteil (30),
- an dem die Kopfkalotte (50) befestigbar ist,
- das am proximalen Ende (12) des Schaftes (10) in verschiedenen Positionen längs einer ersten linearen Richtung befestigbar ist, wobei die erste Richtung in der Resektionsebene oder parallel zu dieser liegt, und
- das an einem Befestigungselement (20) festspannbar ist, das am proximalen Ende (12) des Schaftes (10) in der ersten Richtung einstellbar ist,
**dadurch gekennzeichnet,**
- **dass** das Befestigungselement (20) in einer Führungsbohrung (65) in dem Schaft (10) verschiebbar geführt ist und
- **dass** das Mittelteil durch ein Fenster (66) in der Wand der Führungsbohrung (65) gegen das Befestigungselement (20) spannbar ist, wodurch das Befestigungselement (20) und das Mittelteil (30) fixiert werden.

2. Schulterprothese nach Anspruch 1, wobei die erste Richtung etwa medial-lateral verläuft.

3. Schulterprothese nach Anspruch 1 oder 2, wobei das Mittelteil (30) durch einen Gewindebolzen (40) mit dem Befestigungselement (20) verbindbar und gegen das proximale Ende (12) des Schaftes (10) spannbar ist.

4. Schulterprothese nach Anspruch 1, 2 oder 3, wobei das Mittelteil (30) gegenüber dem Befestigungselement (20) in einer zweiten Richtung einstellbar ist.

5. Schulterprothese nach einem der Ansprüche 1 bis 4, wobei die zweite Richtung etwa rechtwinklig zu der ersten Richtung verläuft, insbesondere etwa anterior-posterior.

6. Schulterprothese nach einem der Ansprüche 1 bis 5, wobei der Gewindebolzen (40) durch eine Langloch-Schulterbohrung (34) im Mittelteil (30) geführt ist.

7. Schulterprothese nach einem der Ansprüche 1 bis 6, wobei die Kopfkalotte (50) exzentrisch an dem Mittelteil (30) befestigbar ist.

8. Schulterprothese nach einem der Ansprüche 1 bis 7, wobei die Kopfkalotte (50) im zusammengebauten Zustand der Endoprothese mit ihrer Unterseite (54) nahezu am proximalen Ende (12) des Schaftes (10) anliegt.

## Claims

1. Shoulder prosthesis
- with a stem (10),
- with a dome shaped head (50) and
- with a middle section (30),
- on which the dome shaped head (50) can be secured,
- which can be secured at the proximal end (12) of the stem (10) in various positions in a first linear direction, whereby the first direction is in the resection plane or parallel to this, and
- that can be tightened on a fastening element (20), that can be adjusted in the first direction at the proximal end (12) of the stem (10),
**characterised in that**
- the fastening element (20) is guided in a guide bore hole (65) in the stem (10) in a displaceable manner and
- **in that** the middle section can be tightened against the fastening element (20) through a window (66) in the wall of the guide bore hole (65), as a result of which the fastening element (20) and the middle section (30) are fixed.

2. Shoulder prosthesis according to Claim 1, in which the first direction is approximately medial-lateral.

3. Shoulder prosthesis according to Claim 1 or 2, in which the middle section (30) can be connected by means of a threaded bolt (40) with the fastening element (20) and tightened against the proximal end (12) of the stem (10).

4. Shoulder prosthesis according to Claim 1, 2 or 3, in which the middle section (30) can be adjusted in relation to the fastening element (20) in a second direction.

5. Shoulder prosthesis according to any one of Claims 1 to 4, in which the second direction is approximately at right-angles to the first direction, in particular approximately anterior-posterior.

6. Shoulder prosthesis according to any one of Claims 1 to 5 in which the threaded bolt (40) is guided through an elongated shouldered bore hole (34) in the middle section (30).

7. Shoulder prosthesis according to any one of Claims 1 to 6, in which the dome shaped head (50) can be secured eccentrically on the middle section (30).

8. Shoulder prosthesis according to any one of Claims 1 to 7, in which the dome shaped head (50) in the assembled state of the endoprosthesis rests with its under side (54) virtually on the proximal end (12) of the stem (10).

## Revendications

1. Prothèse d'épaule comportant
- une tige (10),
- une cupule (50 et
- une partie centrale (30)
- sur laquelle la cupule (50) peut être fixée,
- qui peut être fixée sur l'extrémité proximale (12) de la tige (10) dans différentes positions le long d'une première direction linéaire, la première direction étant située dans le plan de résection ou parallèlement à celui-ci, et
- qui peut être serrée sur un élément de fixation (20) qui peut être réglé sur l'extrémité proximale (12) de la tige (10) dans la première direction,
**caractérisée en ce que**,
- l'élément de fixation (20) peut coulisser dans un alésage de guidage (65) dans la tige (10) et
- **en ce que** la partie centrale peut être fixée contre l'élément de fixation (20) à travers une fenêtre (66) dans la paroi de l'alésage de guidage (65), moyennant quoi l'élément de fixation (20) et la partie centrale (30) sont immobilisés.

2. Prothèse d'épaule selon la revendication 1, la première direction étant approximativement médiane-latérale.

3. Prothèse d'épaule selon la revendication 1 ou 2, la partie centrale (30) pouvant être reliée à l'élément de fixation (20) par un boulon fileté (40) et fixé contre l'extrémité proximale (12) de la tige (10).

4. Prothèse d'épaule selon la revendication 1, 2 ou 3, la partie centrale (30) pouvant être réglée, par rapport à l'élément de fixation (20), dans une deuxième direction.

5. Prothèse d'épaule selon l'une quelconque des revendications 1 à 4, la deuxième direction étant approximativement perpendiculaire à la première direction, en particulier approximativement antérieure-postérieure.

6. Prothèse d'épaule selon l'une quelconque des revendications 1 à 5, le boulon fileté (40) passant à travers un alésage à épaulement oblong (34) dans la partie centrale (30).

7. Prothèse d'épaule selon l'une quelconque des revendications 1 à 6, la cupule (50) pouvant être fixée de façon excentrique sur la partie centrale (30).

8. Prothèse d'épaule selon l'une quelconque des revendications 1 à 7, la cupule (50) ayant sa partie inférieure (54) pratiquement contre l'extrémité proximale (12) de la tige (10) lorsque l'endoprothèse est assemblée.
